Europäisches Patentamt

European Patent Office

Office européen des brevets

(19)

(11) **EP 1 496 121 A2**

(12) **EUROPEAN PATENT APPLICATION**

(43) Date of publication:
    **12.01.2005 Bulletin 2005/02**

(51) Int Cl.⁷: **C12N 15/11**, C07H 21/00,
    A61K 31/7125
    // A61P37/04

(21) Application number: **04023362.9**

(22) Date of filing: **14.08.2000**

(84) Designated Contracting States:
    **AT BE CH CY DE DK ES FI FR GB GR IE IT LI LU
    MC NL PT SE**

(30) Priority: **13.08.1999 US 148798 P**

(62) Document number(s) of the earlier application(s) in
    accordance with Art. 76 EPC:
    **00955527.7 / 1 200 580**

(71) Applicant: **HYBRIDON, INC.
    Cambridge, Massachusetts 02139 (US)**

(72) Inventor: **Agrawal, Sudhir
    Shrewsbury MA 01545 (US)**

(74) Representative: **Grund, Martin, Dr. et al
    Dr. Volker Vossius,
    Patentanwaltskanzlei,
    Geibelstrasse 6
    81679 München (DE)**

Remarks:
    This application was filed on 30 - 09 - 2004 as a
    divisional application to the application mentioned
    under INID code 62.

(54) **Modulation of oligonucleotide CpG-mediated immune stimulation by positional modification of nucleosides**

(57)    The invention provides methods for modulating the immune response caused by CpG-containing oligunucleotides. The methods according to the invention enables both decreasing the immunistimulatory effect for antisense applications, as well as increasing the immunostimulatory effect for immunotherapy applications.

**EP 1 496 121 A2**

**Description**

BACKGROUND OF THE INVENTION

Field of the invention

The invention relates to the therapeutic use of oligonucleotides, both in the antisense approach, and as immunostimulatory agents.

Summary of the related art

Oligonucleotides have become indispensable tools in modern molecular biology, being used in a wide variety of techniques, ranging from diagnostic probing methods to PCR to antisense inhibition of gene expression. This widespread use of oligonucleotides has led to an increasing demand for rapid, inexpensive and efficient methods for synthesizing oligonucleotides.

The synthesis of oligonucleotides for antisense and diagnostic applications can now be routinely accomplished. See e.g., Methods in Molecular Biology, Vol. 20: Protocols for Oligonucleotides and Analogs pp. 165-189 (S. Agrawal, Ed., Humana Press, 1993); Oligonucleotides and Analogues: A Practical Approach, pp. 87-108 (F. Eckstein, Ed., 1991); and Uhlmann and Peyman, supra. Agrawal and Iyer, Curr. Op. in Biotech. 6: 12 (1995); and Antisense Research and Applications (Crooke and Lebleu, Eds., CRC Press, Boca Raton, 1993). Early synthetic approaches included phosphodiester and phosphotriester chemistries. Khorana et al., J. Molec. Biol. 72: 209 (1972) discloses phosphodiester chemistry for oligonucleotide synthesis. Reese, Tetrahedron Lett. 34: 3143-3179 (1978), discloses phosphotriester chemistry for synthesis or oligonucleotides and polynudeotides. These early approaches have largely given way to the more efficient phosphoramidite and H-phosphonate approaches to synthesis. Beaucage and Caruthers, Tetrahedron Lett. 22: 1859-1862 (1981), discloses the use of deoxynucleoside phosphoramidites in polynucleotide synthesis. Agrawal and Zamecnik, U.S. Patent No. 5,149,798 (1992), discloses optimized synthesis of oligonucleotides by the H-phosphonate approach.

Both of these modern approaches have been used to synthesize oligonucleotides having a variety of modified internucleotide linkages. Agrawal and Goodchild, *Tetrahedron Lett.* 28: 3539-3542 (1987), teaches synthesis of oligonucleotide methylphosphonates using phosphoramidite chemistry. Connolly et al., *Biochemistry* 23: 3443 (1984), discloses synthesis of oligonucleotide phosphorothioates using phosphoramidite chemistry. Jager et al., *Biochemistry* 27: 7237 (1988), discloses synthesis of oligonucleotide phosphoramidates using phosphoramidite chemistry. Agrawal et al., *Proc. Natl. Acad. Sci. USA* 85: 7079-7083 (1988), discloses synthesis of oligonucleotide phosphoramidates and phosphorothioates using H-phosphonate chemistry.

More recently, several researchers have demonstrated the validity of the antisense approach to therapeutic treatment of disease. Crooke, Antisense Nucleic Acid Drug Dev. 8: vii-viii, discloses the successful marketing approval of a phosphorothioate oligonucleotide for the treatment of human cytomegalovirus-induced retinitis. Unfortunately, the use of phosphorothioate oligonucleotides has become more complex than originally expected. Certain effects caused by phosphorothioate oligonucleotides could not be explained by the expected antisense mechanism. For example, McIntyre *et al*., Antisense Res. Dev. 3: 309-322 (1993) teaches that a "sense" phosphorothioate oligonucleotide causes specific immune stimulation. This and other side effects have complicated the picture for phosphorothioate oligonucleotides. Zhao *et al*., Biochemical Pharmacology 51:173-182 (1996) discloses immune stimulation mediated by two CpG-containing oligonucleotides, one complementary to the *gag* gene from HIV-1 (5'-CTCTCGCACCCATCTCTCTCCTTCT-3'), and the other complementary to the *rev* gene of HIV-1 (5'-TCGTCGCTGTCTCCGCTTCTTCTTGCC-3').

On the other hand, the observation that phosphodiester and phosphorothioate oligonucleotides can induce immune stimulation has created interest in developing this side effect as a therapeutic tool. These efforts have focussed on phosphorothioate oligonucleotides containing the dinucleotide CpG. Kuramoto *et al*., Jpn. J. Cancer Res. 83: 1128-1131 (1992) teaches that phosphodiester oligonucleotides containing a palindrome that includes a CpG dinucleotide can induce interferon-alpha and gamma synthesis and enhance natural killer activity. Krieg *et al*., Nature 371: 546-549 (1995) discloses that phosphorothioate CpG-containing oligonucleotides are immunostimulatory. Liang *et al*., J. Clin. Invest. 98: 1119-1129 (1996) discloses that such oligonucleotides activate human B cells. Moldoveanu *et al*., Vaccine 16: 1216-124 (1998) teaches that CpG-containing phosphorothioate oligonucleotides enhance immune response against influenza virus. McCluskie and Davis, The Journal of Immunology 161: 4463-4466 (1998) teaches that CpG-containing oligonucleotides act as potent immunostimulatory agents, enhancing immune response against hepatitis B surface antigen. Agrawal, U.S. Patent No. 5,968,909, teaches that backbone modification of C or G suppresses this effect.

These reports make clear that there is a need to be able to modulate the immune response caused by CpG-containing oligonucleotides. Ideally, such modulation should include decreasing the immunostimulatory effect for antisense applications, as well as increasing the immunostimulatory effect for immunotherapy applications.

## BRIEF SUMMARY OF THE INVENTION

The invention provides methods for modulating the immune response caused by CpG-containing oligonucleotides. The methods according to the invention enables both decreasing the immunostimulatory effect for antisense applications, as well as increasing the immunostimulatory effect for immunotherapy applications. Thus, the invention further provides oligonucleotides having optimal levels of immunostimulatory effect for either application and methods for using such oligonucleotides.

The present inventor has surprisingly discovered that positional modification of CpG-containing oligonucleotides dramatically affects their immunostimulatory capabilities. In particular, 2' alkylation or alkoxylation of oligonucleotides at particular positions 5' or 3' to the CpG dinucleotide either enhances or reduces their immunostimulatory effect.

In a first aspect, the invention provides a method for reducing the immunostimulatory effect of a CpG-containing oligonucleotide. The method according to this aspect of the invention comprises introducing a 2' substituted nucleoside into the oligonucleotide at a position adjacent to, and on the 5' side of the CpG dinucleotide, wherein at least one nucleoside is not a 2'-O-methylribonucleoside and the oligonucleotide is not complementary to the *gag* or *rev* gene of human immunodeficiency virus type 1.

In a second aspect, the invention provides a CpG-containing oligonucleotide having a reduced immunostimulatory effect, wherein the oligonucleotide comprises a 2' substituted nucleoside at a position adjacent to, and on the 5' side of the CpG dinucleotide, wherein at least one nucleoside is not a 2'-O-methylribonucleoside and the oligonucleotide is not complementary to the *gag* or *rev* gene of human immunodeficiency virus type 1.

In a third aspect, the invention provides a method for obtaining an antisense-specific reduction in the expression of a gene in a mammal, the method comprising administering to the mammal a CpG-containing oligonucleotide having a reduced immunostimulatory effect, wherein the oligonucleotide comprises a 2' substituted nucleoside at a position adjacent to, and on the 5' side of the CpG dinucleotide, wherein at least one nucleoside is not a 2'-O-methylribonucleoside and the oligonucleotide is not complementary to the *gag* or *rev* gene of human immunodeficiency virus type 1.

In a fourth aspect, the invention provides a method for increasing the immunostimulatory effect of a CpG-containing oligonucleotide, wherein the oligonucleotide is not an antisense oligonucleotide complementary to Ha-ras or the *gag* or *rev* gene of human immunodeficiency virus type 1. The method according to this aspect of the invention comprises introducing into the oligonucleotide a 2' substituted nucleoside at a position selected from the group consisting of 1st nucleoside 5' to the CpG dinucleotide , second nucleoside 5' to the CpG dinucleotide, 3rd nucleoside 5' to the CpG dinucleotide, 4th nucleoside 5' to the CpG dinucleotide, 5th nucleoside 5' to the CpG dinucleotide, 6th nucleoside 5' to the CpG dinucleotide, 2 nucleosides 3' to the CpG dinucleotide, 3rd nucleoside 3' to the CpG dinucleotide, 4th nucleoside 3' to the CpG dinucleotide, 4th nucleoside 3' to the CpG dinucleotide, 4th nucleoside 3' to the CpG dinucleotide, 5th nucleoside 3' to the CpG dinucleotide, 4th nucleoside 3' to the CpG dinucleotide, 6th nucleoside 3' to the CpG dinucleotide, 4th nucleoside 3' to the CpG dinucleotide, and combinations thereof, In certain preferred embodiments, the oligonucleotide is not an antisense oligonucleotide. In alternative embodiments the oligonucleotide may be an antisense oligonucleotide.

In a fifth aspect, the invention provides CpG-containing oligonucleotides having increased immunostimulatory effects, the oligonucleotide comprising a 2' substituted nucleoside at a position selected from the group consisting of 1st nucleoside 5' to the CpG dinucleotide, second nucleoside 5' to the CpG dinucleotide, 3rd nucleoside 5' to the CpG dinucleotide, 4th nucleoside 5' to the CpG dinucleotide, 5th nucleoside 5' to the CpG dinucleotide, 6th nucleoside 5' to the CpG dinucleotide, 2 nucleosides 3' to the CpG dinucleotide, 3rd nucleoside 3' to the CpG dinucleotide, 4th nucleoside 3' to the CpG dinucleotide, 4th nucleoside 3' to the CpG dinucleotide, 4th nucleoside 3' to the CpG dinucleotide, 5th nucleoside 3' to the CpG dinucleotide, 4th nucleoside 3' to the CpG dinucleotide, 6th nucleoside 3' to the CpG dinucleotide, 4th nucleoside 3' to the CpG dinucleotide, and combinations thereof, wherein the oligonucleotide is not an antisense oligonucleotide oligonucleotide complementary to Ha-ras or the *gag* or *rev* gene of human immunodeficiency virus type 1. In certain preferred embodiments, the oligonucleotide is not an antisense oligonucleotide.

In a sixth aspect, the invention provides a method for inducing an immune response in a mammal, the method comprising administering to the mammal an oligonucleotide comprising a 2' substituted nucleoside at a position selected from the group consisting of 1st nucleoside 5' to the CpG dinucleotide , second nucleoside 5' to the CpG dinucleotide, 3rd nucleoside 5' to the CpG dinucleotide, 4th nucleoside 5' to the CpG dinucleotide, 5th nucleoside 5' to the CpG dinucleotide, 6th nucleoside 5' to the CpG dinucleotide, 2 nucleosides 3' to the CpG dinucleotide, 3rd nucleoside 3' to the CpG dinucleotide, 4th nucleoside 3' to the CpG dinucleotide, 4th nucleoside 3' to the CpG dinucleotide, 4th nucleoside 3' to the CpG dinucleotide, 5th nucleoside 3' to the CpG dinucleotide, 4th nucleoside 3' to the CpG dinucleotide, 6th nucleoside 3' to the CpG dinucleotide, 4th nucleoside 3' to the CpG dinucleotide, and combinations thereof, wherein the oligonucleotide is not an antisense oligonucleotide complementary to *Ha-ras* or the *rev* or *gag* gene of human immunodeficiency virus type 1. In certain preferred embodiments, the oligonucleotide is not an antisense oligonucleotide.

## BRIEF DESCRIPTION OF THE DRAWINGS

Figure 1 shows results of a proliferation assay of mouse spleen cells in the presence of no oligonucleotide (C), lipopolysaccharide (LPS), or various oligonucleotides.

Figure 2 shows spleen enlargement in mice administered no oligonucleotide or various oligonucleotides.

Figure 3 shows preferred embodiments of modified bases.

## DETAILED DESCRIPTION OF THE PREFERRED EMBODIMENTS

The invention relates to the therapeutic use of oligonucleotides, both in the antisense approach, and as immunostimulatory agents. The patents and publications cited herein reflect the level of knowledge in the field and are hereby incorporated by reference in their entirety. In the event of conflict between any teaching of any reference cited herein and the present specification, the latter shall prevail, for purposes of the invention.

The invention provides methods for modulating the immune response caused by CpG-containing oligonucleotides. The methods according to the invention enables both decreasing or increasing the immunostimulatory effect for antisense applications, as well as increasing the immunostimulatory effect for immunotherapy applications. Thus, the invention further provides oligonucleotides having optimal levels of immunostimulatory effect for either application and methods for using such oligonucleotides.

The present inventor has surprisingly discovered that positional modification of CpG-containing oligonucleotides dramatically affects their immunostimulatory capabilities. In particular, 2' substitution, including without limitation alkylation or alkoxylation of oligonucleotides at particular positions 5' or 3' to the CpG dinucleotide either enhances or reduces their immunostimulatory effect.

In a first aspect, the invention provides a method for reducing the immunostimulatory effect of a CpG-containing oligonucleotide. The method according to this aspect of the invention comprises introducing a 2' substituted nucleoside into the oligonucleotide at a position adjacent to, and on the 5' side of the CpG dinucleotide, wherein at least one nucleoside is not a 2'-O-methylribonucleoside and the oligonucleotide is not complementary to the *gag* or *rev* gene of human immunodeficiency virus type 1. In preferred embodiments the method is used to make an oligonucleotide that is complementary to a gene or gene transcript. In certain preferred embodiments, the oligonucleotide has antisense activity. In some preferred embodiments, only one 2' substituted nucleoside is introduced into the oligonucleotide for each CpG dinucleotide present in the oligonucleotide. In some preferred embodiments, only one 2' substituted nucleoside is introduced into the oligonucleotide.

As used for the first three aspects of the invention, the term "complementary" means having the ability to hybridize to a genomic region, a gene, or an RNA transcript thereof under physiological conditions. Such hybridization is ordinarily the result of base-specific hydrogen bonding between complementary strands, preferably to form Watson-Crick or Hoogsteen base pairs, although other modes of hydrogen bonding, as well as base stacking can also lead to hybridization. As a practical matter, such hybridization can be inferred from the observation of specific gene expression inhibition.

As used for the first three aspects of this invention, "antisense activity" means that the oligonucleotide, when introduced into a cell or an animal, causes a reduction in the expression of the RNA to which it is complementary.

The method according to this aspect of the invention can be conveniently carried out using any of the well-known synthesis techniques by simply using the appropriate 2' substituted monomer synthon in the synthesis process in the cycle immediately following the incorporation of the CpG dinucleotide. Preferred monomers include phosphoramidites, phosphotriesters and H-phosphonates. Thus, for purposes of the invention, "introducing a 2' substituted nucleoside into the oligonucleotide at a position adjacent to, and on the 5' side of the CpG dinucleotide" simply means synthesizing an oligonucleotide that has a 2' substituted nucleoside at such a position.

In a second aspect, the invention provides a CpG-containing oligonucleotide having a reduced immunostimulatory effect, wherein the oligonucleotide comprises a 2' substituted nucleoside at a position adjacent to, and on the 5' side of the CpG dinucleotide, wherein at least one nucleoside is not a 2'-O-methylribonucleoside and the oligonucleotide is not complementary to the *gag* or *rev* gene of human immunodeficiency virus type 1. Preferably, such oligonucleotides will have from about 12 to about 50 nucleotides, most preferably from about 12 to about 35 nucleotides. Preferred oligonucleotides according to this aspect of the invention are complementary to a gene or gene transcript. More preferably, such oligonucleotides have antisense activity. In some preferred embodiments, the oligonucleotide has only one 2' substituted nucleoside for each CpG dinucleotide present in the oligonucleotide. In some preferred embodiments, the oligonucleotide has only one 2' substituted nucleoside.

In a third aspect, the invention provides a method for obtaining an antisense-specific reduction in the expression of a gene in a mammal, the method comprising administering to the mammal a CpG-containing oligonucleotide having a reduced immunostimulatory effect, wherein the oligonucleotide comprises a 2' substituted nucleoside at a position adjacent to, and on the 5' side of the CpG dinucleotide, wherein at least one nucleoside is not a 2'-O-methylribonucleoside and the oligonucleotide is not complementary to the *gag* or *rev* gene of human immunodeficiency virus type 1. In some preferred embodiments, the oligonucleotide has only one 2' substituted nucleoside for each CpG dinucleotide present in the oligonucleotide. In some preferred embodiments, the oligonucleotide has only one 2' substituted nucleoside.

In the methods according to this aspect of the invention, preferably, administration of oligonucleotides should be parenteral, oral, sublingual, transdermal, topical, intranasal, intravaginal, respiratory, intravitreal, or intrarectal. Administration of the therapeutic compositions can be carried out using known procedures at dosages and for periods of time effective to reduce symptoms or surrogate markers of the disease. When administered systemically, the therapeutic composition is preferably administered at a sufficient dosage to attain a blood level of oligonucleotide from about 0.01 micromolar to about 10 micromolar. For localized administration, much lower concentrations than this may be effective, and much higher concentrations may be tolerated. Preferably, a total dosage of oligonucleotide will range from about 0.01 mg oligonucleotide per patient per day to about 200 mg oligonucleotide per kg body weight per day. The oligonucleotide may be formulated or naked. It may be desirable to administer simultaneously, or sequentially a therapeutically effective amount of one or more of the therapeutic compositions of the invention to an individual as a single treatment episode. In a preferred embodiment, after the composition of matter is administered, one or more measurement is taken of biological effects selected from the group consisting of complement activation, mitogenesis and inhibition of thrombin clot formation.

The method according to this aspect of the invention is useful in animal models of disease or gene expression, and is further useful for the therapeutic treatment of human disease.

In a fourth aspect, the invention provides a method for increasing the immunostimulatory effect of an immunostimulatory motif (*e.g.* CpG)-containing oligonucleotide, wherein the oligonucleotide is not an antisense oligonucleotide complementary to Ha-ras or the *gag* or *rev* gene of human immunodeficiency virus type 1. The method according to this aspect of the invention comprises introducing into the oligonucleotide a 2' substituted nucleoside at a position selected from the group consisting of 1st nucleoside 5' to the CpG dinucleotide , second nucleoside 5' to the CpG dinucleotide, 3rd nucleoside 5' to the CpG dinucleotide, 4th nucleoside 5' to the CpG dinucleotide, 5th nucleoside 5' to the CpG dinucleotide, 6th nucleoside 5' to the CpG dinucleotide, 2 nucleosides 3' to the CpG dinucleotide, 3rd nucleoside 3' to the CpG dinucleotide, 4th nucleoside 3' to the CpG dinucleotide, 4th nucleoside 3' to the CpG dinucleotide, 4th nucleoside 3' to the CpG dinucleotide, 5th nucleoside 3' to the CpG dinucleotide, 4th nucleoside 3' to the CpG dinucleotide, 6th nucleoside 3' to the CpG dinucleotide, 4th nucleoside 3' to the CpG dinucleotide, and combinations thereof In certain preferred embodiments, the oligonucleotide is not an antisense oligonucleotide.

The method according to this aspect of the invention can be conveniently carried out using any of the well-known synthesis techniques by simply using the appropriate 2' substituted monomer synthon in the synthesis process in the cycle immediately following the incorporation of the CpG dinucleotide. Preferred monomers include phosphoramidites, phosphotriesters and H-phosphonates. Thus, for purposes of the invention, "introducing into the oligonucleotide a 2' substituted nucleoside at a position selected from the group consistinglst nucleoside 5' to the CpG dinucleotide , second nucleoside 5' to the CpG dinucleotide, 3rd nucleoside 5' to the CpG dinucleotide, 4th nucleoside 5' to the CpG dinucleotide, 5th nucleoside 5' to the CpG dinucleotide, 6th nucleoside 5' to the CpG dinucleotide, 2 nucleosides 3' to the CpG dinucleotide, 3rd nucleoside 3' to the CpG dinucleotide, 4th nucleoside 3' to the CpG dinucleotide, 4th nucleoside 3' to the CpG dinucleotide, 4th nucleoside 3' to the CpG dinucleotide, 5th nucleoside 3' to the CpG dinucleotide, 4th nucleoside 3' to the CpG dinucleotide, 6th nucleoside 3' to the CpG dinucleotide, 4th nucleoside 3' to the CpG dinucleotide, and combinations thereof" simply means synthesizing an oligonucleotide that has a 2' substituted nucleoside at such a position or positions.

For purposes of the fourth, fifth and sixth aspects of the invention, an "antisense oligonucleotide" is an oligonucleotide that is exactly complementary to a gene or gene transcript, and capable of reducing the expression of the gene or gene transcript to which it is exactly complementary.

In a fifth aspect, the invention provides immunostimulatory motif (e.g. CpG) containing oligonucleotides having increased immunostimulatory effects, the oligonucleotide comprising a 2' substituted nucleoside at a position selected from the group consisting of 1st nucleoside 5' to the CpG dinucleotide, second nucleoside 5' to the CpG dinucleotide, 3rd nucleoside 5' to the CpG dinucleotide, 4th nucleoside 5' to the CpG dinucleotide, 5th nucleoside 5' to the CpG dinucleotide, 6th nucleoside 5' to the CpG dinucleotide, 2 nucleosides 3' to the CpG dinucleotide, 3rd nucleoside 3' to the CpG dinucleotide, 4th nucleoside 3' to the CpG dinucleotide, 4th nucleoside 3' to the CpG dinucleotide, 4th nucleoside 3' to the CpG dinucleotide, 5th nucleoside 3' to the CpG dinucleotide, 4th nucleoside 3' to the CpG dinucleotide, 6th nucleoside 3' to the CpG dinucleotide, 4th nucleoside 3' to the CpG dinucleotide, and combinations thereof, wherein the oligonucleotide is not an antisense oligonucleotide complementary to Ha-ras or the *gag* or *rev* gene of human immunodeficiency virus type 1. In certain preferred embodiments, the oligonucleotide is not an antisense oligonucleotide. Preferred oligonucleotides according to the fourth, fifth and sixth aspects of the invention are from about 6 to about 50 nucleotides in length, and may further comprise modified internucleotide linkages or modified sugars to improve stability.

In a sixth aspect, the invention provides a method for inducing an immune response in a mammal, the method comprising administering to the mammal an immunostimulatory motif (*e.g.* CpG)-containing oligonucleotide comprising a 2' substituted nucleoside at a position selected from the group consisting of 1st nucleoside 5' to the CpG dinucleotide, second nucleoside 5' to the CpG dinucleotide, 3rd nucleoside 5' to the CpG dinucleotide, 4th nucleoside 5' to the CpG dinucleotide, 5th nucleoside 5' to the CpG dinucleotide, 6th nucleoside 5' to the CpG dinucleotide, 2 nucleosides 3' to the CpG dinucleotide, 3rd nucleoside 3' to the CpG dinucleotide, 4th nucleoside 3' to the CpG dinucleotide, 4th nucleoside 3' to the CpG dinucleotide, 4th nucleoside 3' to the CpG dinucleotide, 5th nucleoside 3' to the CpG dinucleotide, 4th nucleoside 3' to the CpG dinucleotide, 6th nucleoside 3' to the CpG dinucleotide, 4th nucleoside 3' to the CpG dinucleotide, and combinations thereof, wherein the oligonucleotide is not an antisense oligonucleotide complementary to *Ha-ras* or the *rev* or *gag* gene of human immunodeficiency virus type 1. In certain preferred embodiments of this aspect of the invention, the oligonucleotide is not an antisense oligonucleotide.

In the methods according to this aspect of the invention, preferably, administration of oligonucleotides should be parenteral, oral, sublingual, transdermal, topical, intranasal, intravitreal, or intrarectal. Administration of the therapeutic compositions can be carried out using known procedures at dosages and for periods of time effective to reduce symptoms or surrogate markers of the disease. When administered systemically, the therapeutic composition is preferably administered at a sufficient dosage to attain a blood level of oligonucleotide from about 0.01 micromolar to about 10 micromolar. For localized administration, much lower concentrations than this may be effective, and much higher concentrations may be tolerated. Preferably, a total dosage of oligonucleotide will range from about 0.01 mg oligonucleotide per patient per day to about 200 mg oligonucleotide per kg body weight per day. It may be desirable to administer simultaneously, or sequentially a therapeutically effective amount of one or more of the therapeutic compositions of the invention to an individual as a single treatment episode. In a preferred embodiment, after the composition of matter is administered, one or more measurement is taken of biological effects selected from the group consisting of IL-12 induction, and immune cell mitogenesis. The. oligonucleotides may be administered alone, or in combination with specific antigens, which may or may not be physically attached to the oligonucleotide. Such administration may optionally include the use of adjuvants.

The method according to this aspect of the invention is useful for model studies of the immune system, and is further useful for the therapeutic treatment of human disease.

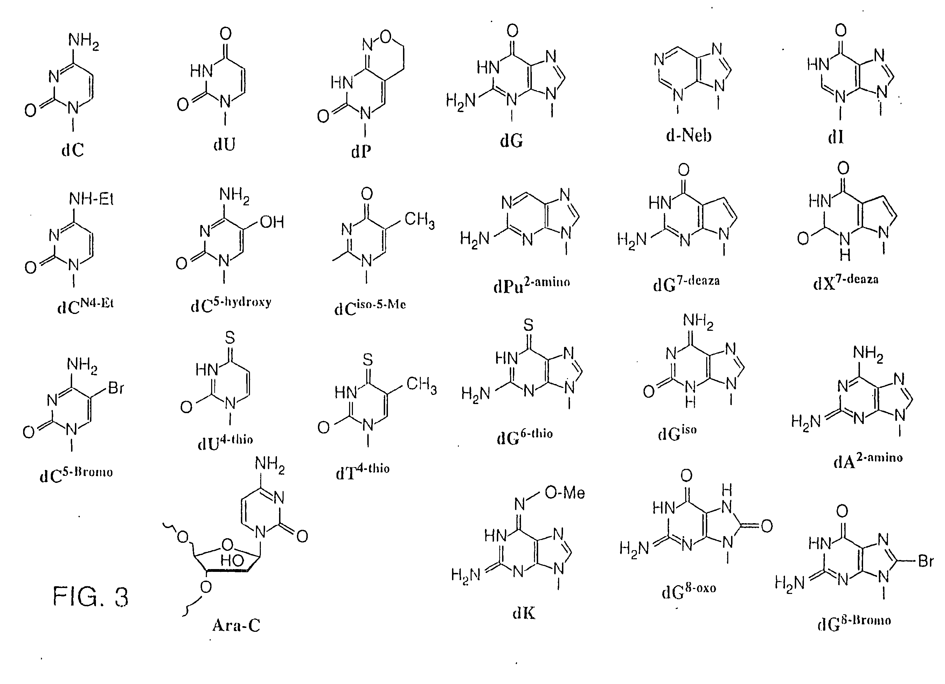

For purposes of all aspects of the invention, the term "oligonucleotide" includes polymers of two or more deoxyribonucleotide, or any modified nucleoside, including 2'-halo-nucleosides, 2'-O-substitutednucleosides ribonucleosides, deazanucleosides or any combination thereof. Such monomers may be coupled to each other by any of the numerous known internucleoside linkages. In certain preferred embodiments, these internucleoside linkages may be nonionic, boronic, phosphodiester, phosphotriester, phosphorothioate, or phosphoramidate linkages, 2'-5' , 3'-5', 3'-3'linkages of any of the forgoing, or combinations thereof. The term oligonucleotide also encompasses such polymers having chemically modified bases or sugars and/or having additional substituents, including without limitation lipophilic groups, intercalating agents, diamines and adamantane. For purposes of the invention the term "2'-O-substituted" means substitution of the 2' position of the pentose moiety with a halogen (preferably Cl, Br, or F), or an -O-lower alkyl group containing 1-6 saturated or unsaturated carbon atoms, or with an -O-aryl or allyl group having 2-6 carbon atoms, wherein such alkyl, aryl or allyl group may be unsubstituted or may be substituted, e.g., with halo, hydroxy, trifluoromethyl, cyano, nitro, acyl, acyloxy, alkoxy, carboxyl, carbalkoxyl, or amino groups; or such 2' substitution may be with a hydroxy group (to produce a ribonucleoside), an amino or a halo group, but not with a 2'-H group. Such oligonucleotides include oligonucleotides having modified sugars (*e.g.* arabinose, hexose) and other backbone modifications, *e.g.*, as in peptide nucleic acid and locked nucleic acid. Such oligonucleotides may also have naturally occurring bases or modified heterocyclic rings, including without limitation those shown in Figure 3. For purposes of all aspects of the invention, the terms "CpG" or "CpG dinucleotide" means the dinucleotide 5'-cytidine-guanidine-3', wherein p is an internucleotide linkage, and wherein the sugar backbone of the dinucleotide deoxyribose, or a modified sugar, or combinations thereof. In preferred embodiments of the first three aspects of the invention, p is selected from phosphodiester, phosphorothioate, phosphorodithioate, stereospecific (Rp or Sp) phosphorothioate covalent linkages of any of the above. The non-phosphodiester, non-phosphorothioate embodiments will further reduce immunostimulatory effects. In preferred embodiments of the last three aspects of the invention, p is selected from phosphodiester, phosphorothioate and phosphordithioate.

The following examples are intended to further illustrate certain preferred embodiments of the invention, and are not intended to limit the scope of the invention.

Example 1

Modulation of immulostimulatory effect in vitro

To study the impact of site of chemical modification of PS-oligos containing CpG motif, we chose two oligonucleotides. Oligo 1, which contains one CpG motif and Oligo 2, which contains two CpG motifs. Both of these oligos have been studied earlier and have shown to be immunostimulatory. To evaluate the immunostimulatory activity of oligonucleotides in the present study, we have used mouse spleen cell proliferation assay.

Table 1 Oligodeoxynucleotide Phosphorothioates and Site of Modification:

| Oligo No. | Sequence & Modification (5'-3') | |
|---|---|---|
| 1 | T C C A T G A C G T T C C T G A T G C | |
| 2 | T C C A T **G A** C G T T C C T G A T G C | |
| 3 | T C C A **U G** A C G T T C C T G A T G C | |
| 4 | T C C **A U** G A C G T T C C T G A T G C | |
| 5 | T C C **A** T G A C G T T C C T G A T G C | |
| 6 | T C C A T G A C G **U U** C C T G A T G C | |
| 7 | T C C A T G A C G T **U C** C T G A T G C | |
| 8 | T C C A T G A C G T T **C C** T G A T G C | |
| 9 | T C C A T G A C G T T C C **U** G A T G C | |
| 10 | T C C A T G A C G T T C C **U G** A T G C | |
| 11 | T C C A T G A C G T T C C T **G A** T G C | |
| 12 | T C C **A** T G A C G T T C C **U** G A T G C | |
| 13 | T C C A T G A C G T T C C T G **A U G C** | |
| 14 | T C C **A** T G A C G T T C C T G **A U G C** | |
| 15 | T C C A T G A C G T T C C T G A C G T T | |
| 16 | T C C **A U** G A C G T T C C T G A C G T T | |
| 17 | T C C A T G A C G T T C **C U** G A C G T T | |
| 18 | T C C **A U** G A C G T T C **C U** G A C G T T | |

Bold face

Normal face

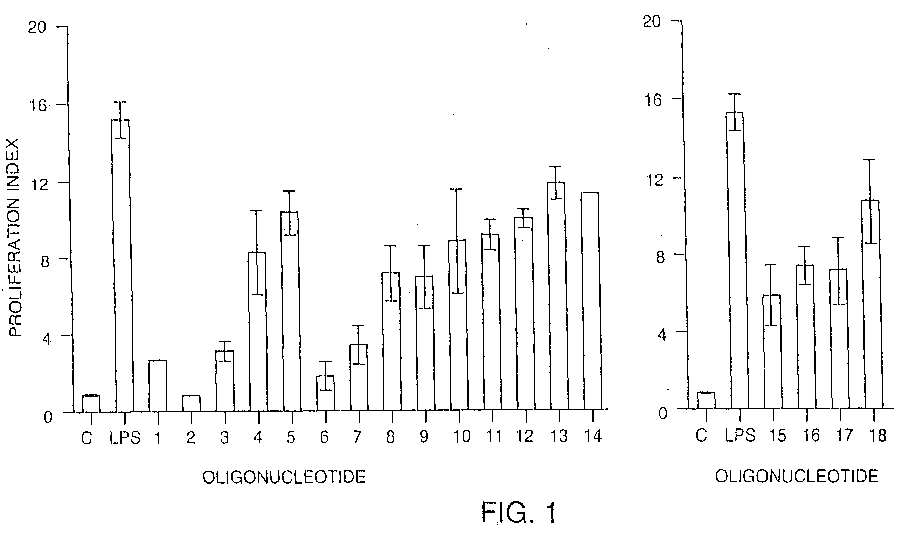

Mouse spleen lymphocytes were cultured with oligonucleotides at concentration of 0.1, 1, and 10 μg/mL. Oligo 1 induced a dose dependent effect on cell proliferation. At 0.1 μg/mL, the proliferation index was 287 (Fig. 1). Substitution of 5'-flanking GA deoxynucleosides of CpG motif of Oligo 1 with 2'-OMe, (oligo 2), resulted in complete suppression of cell proliferation at all concentrations used (Fig 1). At 0.1 μg/mL, cell proliferation index was similar to medium alone. Substitution of the 3'- flanking TT deoxynucleosides of CpG motif of Oligo 1 with 2'-OMe (Oligo 6) did not have such an impact on cell proliferation. The proliferation index with Oligo 6 was 2.07 (Fig. 1). To further understand if substitution of two deoxynucleosides in the 5'-flanking region with 2'-OMe away from the CpG motif would have any impact on induced cell proliferation, we synthesized oligos 3, 4 and 5 (Table 1). Two deoxynucleosides were substituted with 2'-OMe leaving one, two and three deoxynucleosides respectively in between site of substitution and CpG motif. The proliferation index of oligo 3, 4, and 5 was 3.42, 8.44, and 10.38 respectively which is an increase of 29, 297 and 400 percent, respectively, compared to oligo 1 (Fig.1). Substitution of remaining deoxynucleosides further towards 5'-end than in oligo 5 showed no further increase in proliferation index (data not shown).

Similar substitutions were made in oligo 1 in the 3'- flanking region to CpG motif. Oligo **7, 8, 9, 10** and **11** were synthesized in which two deoxynucleosides were substituted with 2'-OMe leaving one, two, three, four and five deoxynucleosides respectively in between CpG motif and 2'-OMe substitution. The proliferation index of oligo **7, 8, 9, 10** and **11** were 3.63, 7.22, 7.01, 8.85, and 9.24 respectively. Compared to oligo 1, the increase in proliferation index for oligo **7, 8, 9, 10** and **11** was 39, 231, 221, 317 and 338 percent respectively.

From these results, it is evident that substitution of two deoxynucleotides at either the 5'-end or the 3'-end of the CpG motif (e.g. Oligo 5 or 9) increases the immunostimulatory activity. Is it possible that the substitution made in Oligo **5** and Oligo **9** will have additive effects in further increasing the immunostimulatory activity? We synthesized oligo **12**, which had two deoxynucleosides that were substituted with 2'-OMe at both the 3'-end and the 5'-end. Oligo **12** did not show further increase in the proliferation index when compared with Oligo 5, however, it had increased proliferation index compared to Oligo **9**.

To explore if the above observations made with the use of Oligos 3 to 5 and Oligos 7 to 11 are sequence specific or general, we made the same modifications to Oligo 15, which contains two CpG motifs. Oligo 15 had a proliferation index of 5.83 at a concentration of 0.1 g/mL. Substitution of two deoxynucleosides at 5'- ends of individual CpG motifs leaving two deoxynucleosides in between CpG motif and substitution with 2'-OMe, oligo **16** and **17,** showed proliferation index of 7.34 and 7.13 respectively, which is only an increase of twenty percent compared to oligo 15. Substitution of two deoxynucleosides in the 5'- flanking region of both CpG motifs (Oligo **18**) showed a higher proliferation index 10.62, which is an increase of about fifty percent compared to Oligo **15**.

Example 2

Effect of nuclease stability on immunostimulatory activity

In addition to site specific substitution, we also questioned if increased metabolic stability of PS-oligo containing CpG motif may result in increased cell proliferation and if that can be combined with 5'-substitutions to further increase the cell proliferation activity. Oligo 13 was synthesized in which four continuous deoxynucleosides at the 3'-end of oligo 1 were substituted with 2'-OMe, which results in a significant increase in stability towards nucleases. Oligo 13 had a proliferation index of 11.92, which is an increase of about 480% compared to oligo 1 (Fig. 1). Further modification of oligo 13 by substitution of two deoxynucleosides at 5'-end (oligo 14) did not result in further increase in proliferation index.

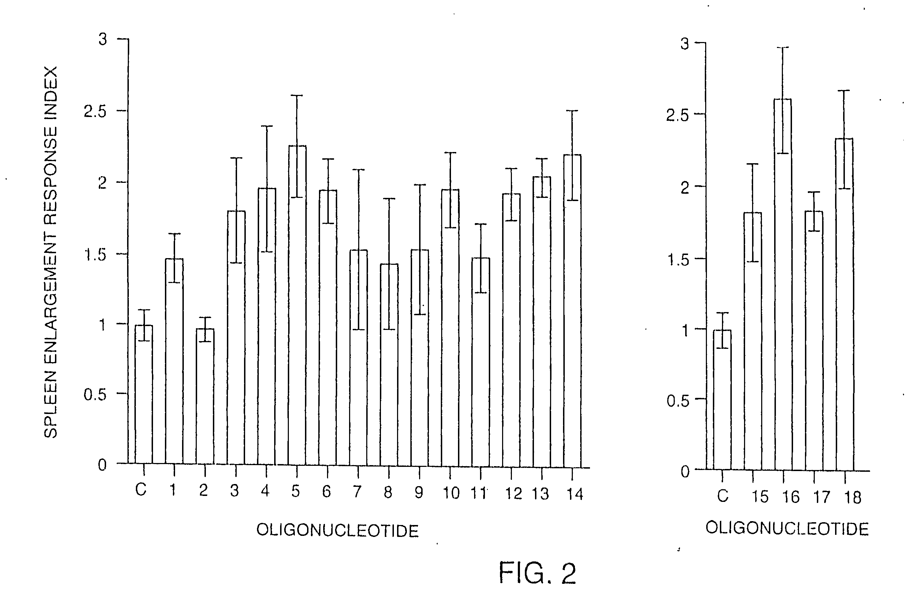

After observing that above substitutions in PS-oligos modulates its immunostimulatory activity based on cell culture assay, we administered oligonucleotides listed in Table 1 intraperitonealy to mice and measured the spleen weights to confirm that if substitutions have same effect in vivo. Administration of oligo 1 caused about 50 percent increase in spleen weight (Fig. 2). Oligo 2, which had shown no immunostimulatory activity in cell culture assay, showed no increase in spleen weights (Fig. 2). Substitution of two deoxynucleotides away from CpG motif towards 5'-end, oligo 3, 4, and 5 showed progressive increase in spleen weights which were 67, 95 and 157 percent respectively more compared to mice treated with oligo 1 (Fig. 2), confirming an increase in their immunostimulatory activity. Substitutions of two deoxynucleosides with 2'-OMe toward the 3'-end of the CpG motif, in general, had less significant increase in spleen weight. Only oligo 6 and 11 caused an increase in spleen weight of 97 and 95 percent compared to oligo **1**. Oligo **12**, which had substitutions made at both the 3'-end and the 5'-end showed 95 percent increase in spleen weight compared to oligo 1 (Fig. 2).

Oligos **15, 16, 17**, and **18**, all of which contain two CpG motifs, showed increase in spleen weight following administration of a dose of 5 mg/kg. Oligo **15** caused 175 percent increase of spleen weight compared to untreated mice. Oligo **16,** in which two of the 5'-end CpG motifs were substituted with 2'-OMe, caused a 93 percent increase in spleen weight compared to oligo **15** (Fig. 2). Oligo **17**, which has substitutions at the 5'-end of one CpG (but at the 3'-end of the other CpG motif) showed no further increase in spleen weight. Substitution at the 5'-end of both CpG motifs did not result in further increases in spleen weight compared to oligo 16, however had only about 60 percent increase over oligo 15 (Fig. 2).

Oligo **13**, which is metabolically stable compared to oligo **1**, showed 114 percent increase in spleen weight compared to Oligo **1**, and was in agreement with cell proliferation data. Similar results were observed with Oligo **14**, which has increased metabolic stability and substituted at the 5'-end of CpG motif (Fig. 2).

**Claims**

1. A method for reducing the immunostimulatory effect of a CpG-containing oligonucleotide, the method comprising introducing a 2' substituted nucleoside into the oligonucleotide at a position adjacent to, and on the 5' side of the CpG dinucleotide, wherein at least one nucleoside is not a 2'-O-methylribonucleoside and the oligonucleotide is not complementary to the *gag* or *rev* gene of human immunodeficiency virus type 1.

2. The method according to claim 1, wherein the method is used to make an oligonucleotide that is complementary to a gene or gene transcript.

3. The method according to claim 2, wherein the oligonucleotide has antisense activity.

4. The method according to claim 1, wherein only one 2' substituted nucleoside is introduced into the oligonucleotide for each CpG dinucleotide present in the oligonucleotide.

5. The method according to claim 1, wherein only one 2' substituted nucleoside is introduced into the oligonucleotide.

6. A CpG-containing oligonucleotide having a reduced immunostimulatory effect, wherein the oligonucleotide consisting of at least one 2' substituted nucleoside at a position adjacent to, and on the 5' side of the CpG dinucleotide, wherein at least one nucleoside is not a 2'-O-methylribonucleoside and the oligonucleotide is not complementary to the *gag* or *rev* gene of human immunodeficiency virus type 1.

7. The oligonucleotide according to claim 6, wherein the oligonucleotide is from about 8 to about 50 nucleotides in length.

8. The oligonucleotide according to claim 7, wherein the oligonucleotide is from about 12 to about 35 nucleotides in length.

9. The oligonucleotide according to claim 6, wherein the oligonucleotide is complementary to a gene or gene transcript.

10. The oligonucleotide according to claim 6, wherein the oligonucleotide has antisense activity.

11. The oligonucleotide according to claim 6, wherein the oligonucleotide has only one 2' substituted nucleoside for each CpG dinucleotide present in the oligonucleotide.

12. The oligonucleotide according to claim 7, wherein the oligonucleotide has only one 2' substituted nucleoside.

13. A composition of pharmaceutical use comprising a CpG-containing oligonucleotide having a reduced immunostimulatory effect, wherein the oligonucleotide comprises a 2' substituted nucleoside at a postion adjacent to, and on the 5' side of the CpG dinucleotide, wherein at least one nucleoside is not a 2'-O-methylribonucleoside and the oligonucleotide is not complementary to the *gag* or *rev* gene of human immunodeficiency virus type 1.

14. The composition according to claim 13, wherein the oligonucleotide has only one 2' substituted nucleoside for each CpG dinucleotide present in the oligonucleotide.

15. The composition according to claim 13, wherein the oligonucleotide has only one 2' substituted nucleoside.

FIG. 1

FIG. 2

FIG. 3

SEQUENCE LISTING

<110> HYBRIDON, INC.

<120> MODULATION OF OLIGONUCLEOTIDE
      CpG-MEDIATED IMMUNE STIMULATION BY POSITIONAL MODIFICATION
      OF NUCLEOSIDES

<130> 115-84 T1

<140> EP 04 023 362.9
<141> 2000-08-14

<150> PCT/US00/22284
<151> 2000-08-14

<150> 60/148,798
<151> 1999-08-13

<160> 20

<170> FastSEQ for Windows Version 4.0

<210> 1
<211> 25
<212> DNA
<213> Artificial Sequence

<220>
<223> Oligonucleotide  complementary to gag gene of
      HIV-1

<400> 1
ctctcgcacc catctctctc cttct                                        25

<210> 2
<211> 27
<212> DNA
<213> Artificial Sequence

<220>
<223> Oligonucleotide  complementary to rev gene of
      HIV-1

<400> 2
tcgtcgctgt ctccgcttct tcttgcc                                      27

<210> 3
<211> 19
<212> DNA
<213> Artificial Sequence

<220>
<223> Oligodeoxynucleotide phosphorothioates

<400> 3

```
tccatgacgt tcctgatgc                                                    19

<210> 4

<211> 19
<212> DNA
<213> Artificial Sequence

<220>
<223> Oligodeoxynucleotide phosphorothioates

<400> 4
tccatgacgt tcctgatgc                                                    19

<210> 5
<211> 19
<212> DNA
<213> Artificial Sequence

<220>
<223> Oligodeoxynucleotide phosphorothioates

<400> 5
tccaugacgt tcctgatgc                                                    19

<210> 6
<211> 19
<212> DNA
<213> Artificial Sequence

<220>
<223> Oligodeoxynucleotide phosphorothioates

<400> 6
tccaugacgt tcctgatgc                                                    19

<210> 7
<211> 19
<212> DNA
<213> Artificial Sequence

<220>
<223> Oligodeoxynucleotide phosphorothioates

<400> 7
tccatgacgt tcctgatgc                                                    19

<210> 8
<211> 19
<212> DNA
<213> Artificial Sequence

<220>
<223> Oligodeoxynucleotide phosphorothioates

<400> 8
tccatgacgu ucctgatgc                                                    19
```

```
<210> 9
<211> 19
<212> DNA
<213> Artificial Sequence

<220>
<223> Oligodeoxynucleotide phosphorothioates

<400> 9
tccatgacgt ucctgatgc                                                    19


<210> 10
<211> 19
<212> DNA
<213> Artificial Sequence

<220>
<223> Oligodeoxynucleotide phosphorothioates

<400> 10
tccatgacgt tcctgatgc                                                    19


<210> 11
<211> 19
<212> DNA
<213> Artificial Sequence

<220>
<223> Oligodeoxynucleotide phosphorothioates

<400> 11
tccatgacgt tccugatgc                                                    19


<210> 12
<211> 19
<212> DNA
<213> Artificial Sequence

<220>
<223> Oligodeoxynucleotide phosphorothioates

<400> 12
tccatgacgt tccugatgc                                                    19


<210> 13
<211> 19
<212> DNA
<213> Artificial Sequence

<220>
<223> Oligodeoxynucleotide phosphorothioates

<400> 13
tccatgacgt tcctgatgc                                                    19


<210> 14
<211> 19
<212> DNA
```

<210> Artificial Sequence

<220>
<223> Oligodeoxynucleotide phosphorothioates

<400> 14
tccatgacgt tccugatgc                                                    19

<210> 15
<211> 19
<212> DNA
<213> Artificial Sequence

<220>
<223> Oligodeoxynucleotide phosphorothioates

<400> 15
tccatgacgt tcctgaugc                                                    19

<210> 16
<211> 19
<212> DNA
<213> Artificial Sequence

<220>
<223> Oligodeoxynucleotide phosphorothioates

<400> 16
tccatgacgt tcctgaugc                                                    19

<210> 17
<211> 20
<212> DNA
<213> Artificial Sequence

<220>
<223> Oligodeoxynucleotide phosphorothioates

<400> 17
tccatgacgt tcctgacgtt                                                   20

<210> 18
<211> 20
<212> DNA
<213> Artificial Sequence

<220>
<223> Oligodeoxynucleotide phosphorothioates

<400> 18
tccaugacgt tcctgacgtt                                                   20

<210> 19
<211> 20
<212> DNA
<213> Artificial Sequence

<220>

```
<223> Oligodeoxynucleotide phosphorothioates

<400> 19
tccatgacgt tccugacgtt                                              20


<210> 20
<211> 20
<212> DNA
<213> Artificial Sequence

<220>
<223> Oligodeoxynucleotide phosphorothioates

<400> 20
tccaugacgt tccugacgtt                                              20
```